# EUROPEAN PATENT SPECIFICATION

(11) **EP 3 763 817 B1**
(45) Date of publication and mention of the grant of the patent: **28.10.2026**
(21) Application number: 19763648.3
(22) Date of filing: 08.03.2019
(51) Int. Cl.: C12N 9/02, C07K 14/245, C12N 9/04, C12N 11/02, C12N 15/70, C12Q 1/00, C12Q 1/32

(54) **FUSION PROTEIN OF FLAVIN ADENINE DINUCLEOTIDE-GLUCOSE DEHYDROGENASE AND CYTOCHROME MOLECULE**
FUSIONSPROTEIN VON FLAVINADENIN-DINUKLEOTID-GLUCOSE-DEHYDROGENASE UND CYTOCHROMMOLEKÜL
PROTÉINE DE FUSION DE FLAVINE ADÉNINE DINUCLÉOTIDE-GLUCOSE DÉSHYDROGÉNASE ET MOLÉCULE CYTOCHROME

(30) Priority: 08.03.2018 JP 2018042275
(43) Date of publication of application: 13.01.2021
(73) Proprietor: i-Sens, Inc., Seoul 06646 (KR)
(72) Inventor: SODE, Koji, Tokyo 183-8538 (JP); KANG, Youngjea, Seoul 06646 (KR); MORI, Kazushige, Tokyo 152-0013 (JP); KOJIMA, Katsuhiro, Tokyo 152-0013 (JP); SHIMAZAKI, Junko, Tokyo 152-0013 (JP)
(74) Representative: Brann AB
(86) International application number: PCT/JP2019/009235
(87) International publication number: WO 2019/172400

(56) References cited:
- EP-A1- 2 589 659
- WO-A1-2010/126139
- WO-A1-2017/094776
- JP-A- 2001 518 798
- JP-A- 2002 515 253
- JP-A- H08 275 780
- ALGOV ITAY ET AL: "Highly Efficient Flavin-Adenine Dinucleotide Glucose Dehydrogenase Fused to a Minimal Cytochrome C Domain", vol. 139, no. 48, 6 December 2017 (2017-12-06), pages 17217 - 17220, XP055853359, ISSN: 0002-7863, Retrieved from the Internet <URL:https://pubs.acs.org/doi/pdf/10.1021/jacs.7b07011> DOI: 10.1021/jacs.7b07011
- ITO KOHEI ET AL: "Designer fungus FAD glucose dehydrogenase capable of direct electron transfer", BIOSENSORS AND BIOELECTRONICS, ELSEVIER SCIENCE LTD, UK, AMSTERDAM , NL, vol. 123, 26 July 2018 (2018-07-26), pages 114 - 123, XP085514329, ISSN: 0956-5663, DOI: 10.1016/J.BIOS.2018.07.027
- K ISHIDA ET AL: "Comparison of Direct and Mediated Electron Transfer in Electrodes with Novel Fungal Flavin Adenine Dinucleotide Glucose Dehydrogenase", ANAL. SCI., vol. 34, no. 7, 1 July 2018 (2018-07-01), pages 783 - 787, XP055653326, DOI: 10.2116/analsci.17P613

## Description

### TECHNICAL FIELD

The present disclosure relates to a fusion protein comprising a fungus-derived FADGDH and a cytochrome molecule linked to the N terminus of the FADGDH.

### BACKGROUND ART

In the treatment of diabetes, there is a case in which a diabetic patient needs to control his or her blood glucose level (self-monitoring blood glucose (SMBG)). Nowadays, SMBG has become an important health management method in the treatment of diabetes.

Nowadays, a variety of SMBG methods have been developed and reported. Examples thereof include a method in which an enzyme that uses blood glucose as a substrate is used as a sensing element. According to this method, the sensing element enzyme catalyzes a reaction using blood glucose as a substrate, and the resulting electrons are transferred to an electrode through an electron mediator, thereby generating a current. The level of glucose in the blood may be quantified by measuring the magnitude of the current. Enzymes that are used as sensing elements include glucose oxidase, pyrroloquinoline quinone glucose dehydrogenase (PQQGDH), flavin adenine dinucleotide glucose dehydrogenase (FADGDH), and the like (Patent Documents 1 and 2) .

In addition, the use of a fusion protein including the enzyme and a cytochrome molecule as a sensing element has also been reported and reviewed.

Patent Document 3 reported constructing a modified PQQGDH by linking the cytochrome c domain of PQQ ethanol dehydrogenase (PQQEDH), which uses PQQ as a coenzyme, at the gene level to the carboxyl terminus of the PQQGDH, and reported that the fusion protein had direct electron transfer (DET) ability. In addition, Non-Patent Document 1 reported that a fusion protein obtained by connecting the bacteria-derived cytochrome c to the C-terminus of the catalytic subunit of the bacteria-derived FADGDH has DET ability.

EP2589659 discloses a fusion product comprising a secreted extracellular cytochrome and a flavine-dinucleotide-dependent glucose dehydrogenase (FADGDH)for use in glucose measurement.

WO2017094776 discloses a cytochrome b-glucose dehydrogenase fused protein having modified electron transfer characteristics for use in measuring glucose.

Algov Itay et al. ("Highly efficient Flavin-Adenine Dinucleotide Glucose Dehydrogenase Fused to a Minimal Cytochrome C Domain", Journal of the American Chemical Society, vol.139, no.48, 6 December 2017, pages 17217-17220) disclose a fusion enzyme comprising a FADGDH from *Burkholderia cepacia* fused to a minimal cytochrome C domain for use in blood glucose monitoring.

In addition, an attempt to link a cytochrome molecule to a specific fusion protein comprising a fungus-derived FADGDH according to the present invention has not yet been reported.

### Prior Art Documents

### Patent Documents

Patent Document 1: Japanese Patent Application Laid-Open No. 2012-210212
Patent Document 2: WO2009/037838
Patent Document 3: Japanese Patent Application Laid-Open No. 2014-207911

### Non-Patent Documents

Non-Patent Document 1: J. Am. Chem. Soc., 2017, 139(48), pp. 17217-17220

### DISCLOSURE

### Technical Problem

In a fungus-derived FADGDH, the coenzyme FAD is buried deep in the protein molecule. For this reason, in the absence of an external electron acceptor (mediator), FAD reduced by oxidation (dehydrogenation) of glucose cannot be oxidized by an electrode to obtain electrons. With the aim of achieving the above objective, it has been attempted to impart DET ability to fungus-derived FADGDHs by elaborately modifying carbon nanotubes or gold nanoparticles on electrodes or enzymes. However, since there is an error in the interpretation of the obtained phenomenon or a complex chemical modification process is required, good results have not been obtained until now.

Furthermore, GDH or EDH using the above-described PQQ as a coenzyme has a completely different structure from a fungus-derived GDH. In addition, for a fusion protein comprising PQQEDH and a cytochrome molecule, the molecular surface, to which the coenzyme PQQ is bound, has a structure in which the Si side is widely open toward PQQ. For this reason, it is considered that the cytochrome molecule is particularly easy to access, and the fusion protein exhibits DET ability due to the above-described structure.

In addition, for the fusion protein comprising a bacteria-derived FADGDH and a bacteria-derived cytochrome c, iron sulfur clusters present in the bacteria-derived FADGDH accept electrons from FAD, and furthermore, transfer the accepted electrons to cytochrome c or other external electron acceptors (Bio electro chemistry Volume 112, December 2016, Pages 178-183). That is, it is considered that the DET ability in the fusion protein is based on the iron sulfur clusters in the bacteria-derived FADGDH, and is a unique phenomenon based on the enzyme. In addition, it is considered that the bacteria-derived FADGDH has a remarkably different primary structure from the fungus-derived FADGDH, and that the three-dimensional structures of the two proteins differ from each other.

From the above, a fusion protein comprising a fungus-derived FADGDH according to claim 1 having direct electron transfer ability has not yet been obtained. Therefore, an objective of the present disclosure is to provide such a fungus-derived FADGDH having direct electron transfer ability.

### Technical Solution

The present inventors have worked hard to solve the above problem, and as a result, have found that a fusion protein according to claim 1 comprising a fungus-derived flavin adenine dinucleotide glucose dehydrogenase (FADGDH) and a cytochrome molecule linked to the N-terminus of the FADGDH enables direct electron transfer without requiring an electron mediator.

The present disclosure is based on the above understanding, and includes the following inventions.
[1] A fusion protein comprising:
   (a) a protein which consists of the amino acid sequence set forth in at least one of SEQ ID NOs: 2, 3 and 13 to 21;
      and
   (b) a protein which consists of an amino acid sequence having a sequence identity of 85% or higher to the amino acid sequence (a), and which has glucose dehydrogenase activity and electron transfer function.
[2] A gene encoding the fusion protein of [1].
[3] A vector comprising the gene of [2].
[4] A transformant comprising the gene of [2].
[5] A transformant in which the gene of [2] is integrated into the main chromosome of the transformant.
[6] An enzyme electrode comprising the fusion protein of [1] attached thereto.
[7] A method for measuring glucose concentration in a sample, the method comprising:
   bringing the sample into contact with the enzyme electrode of [6]; and
   measuring electrons generated by oxidation of glucose.
[8] A glucose sensor which uses the enzyme electrode of [6] as a working electrode.

### Advantageous Effects

According to the present disclosure, there may be provided a fungus-derived FADGDH having direct electron transfer ability according to claim 1.

### DESCRIPTION OF DRAWINGS

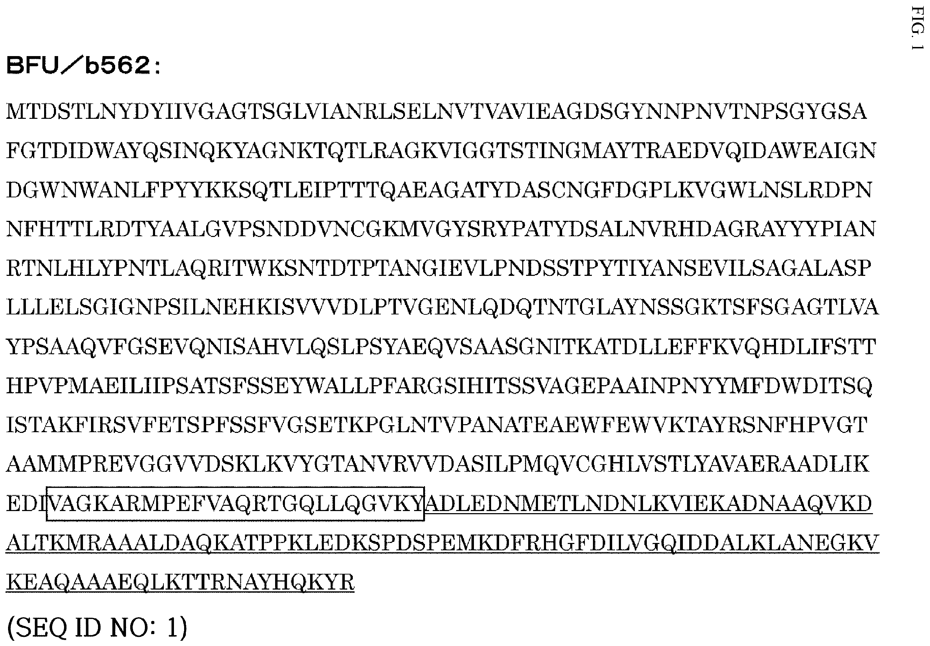

FIG. 1 illustrates the amino acid sequence (SEQ ID NO: 1) of a fusion protein (BFU/b562) obtained by linking *E*. coli-derived cytochrome b562 (b562) to the C-terminus of a fungus-derived FADGDH (BFU) via a linker sequence. The boxed sequence indicates the linker sequence, and the underlined portion indicates the sequence of b562.

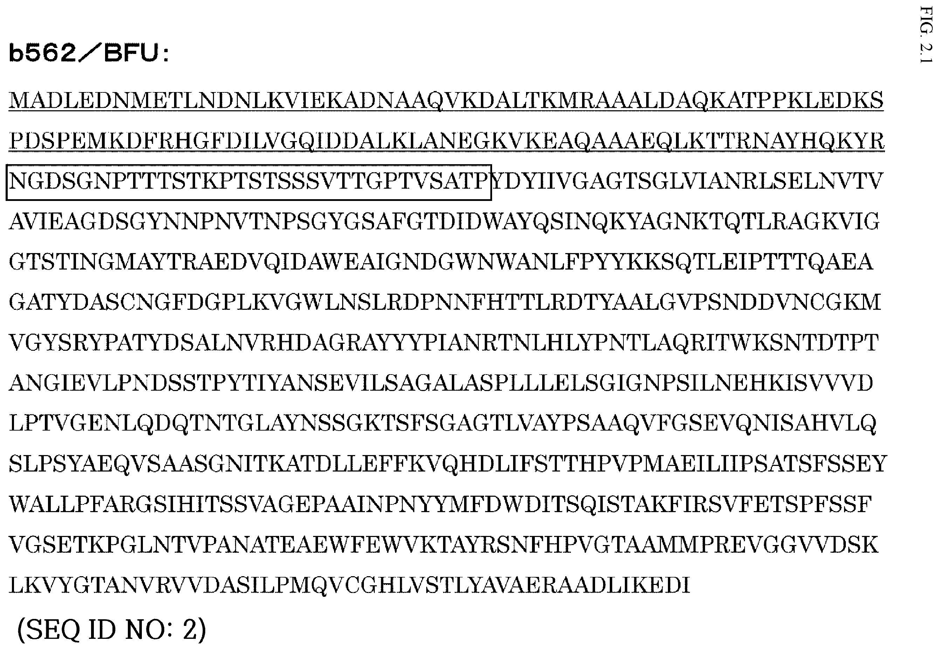

FIG. 2-1 illustrates the amino acid sequence (SEQ ID NO: 2) of a fusion protein (b562/BFU) obtained by linking *E*. coli-derived cytochrome b562 (b562) to the N-terminus of a fungus-derived FADGDH (BFU) via a linker sequence. The boxed sequence indicates the linker sequence, and the underlined portion indicates the sequence of b562.

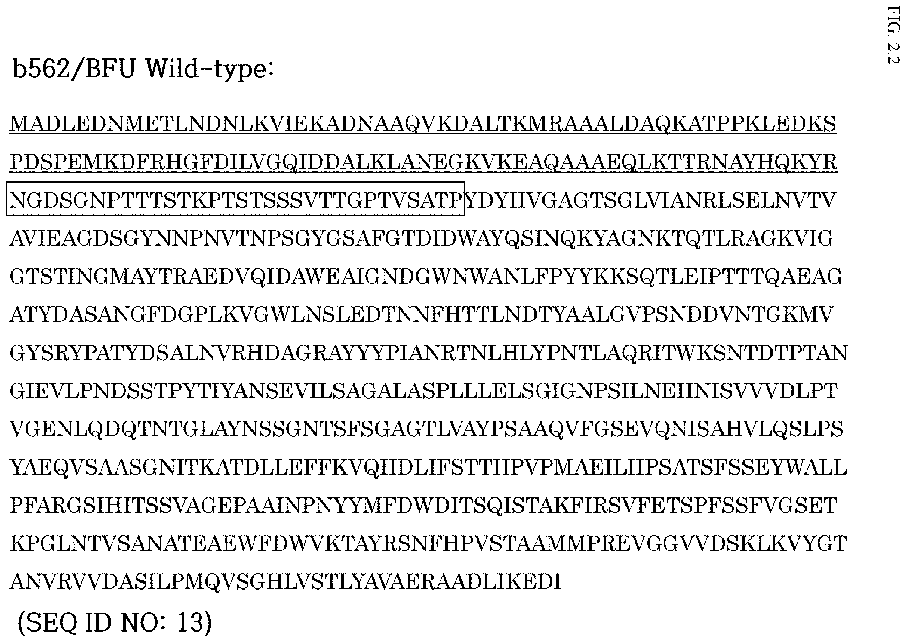

FIG. 2-2 illustrates the amino acid sequence (SEQ ID NO: 13) of a fusion protein (b562/BFU wild-type) obtained by linking *E*. coli-derived cytochrome b562 (b562) to the N-terminus of a fungus-derived FADGDH (BFU wild-type) via a linker sequence. The boxed sequence indicates the linker sequence, and the underlined portion indicates the sequence of b562.

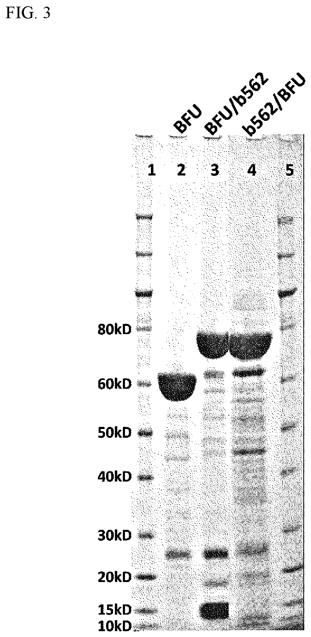

FIG. 3 is a photograph illustrating the results of SDS-PAGE analysis of the fungus-derived FADGDH (BFU), fusion protein (BFU/b562) and fusion protein (b562/BFU) expressed in transformants.

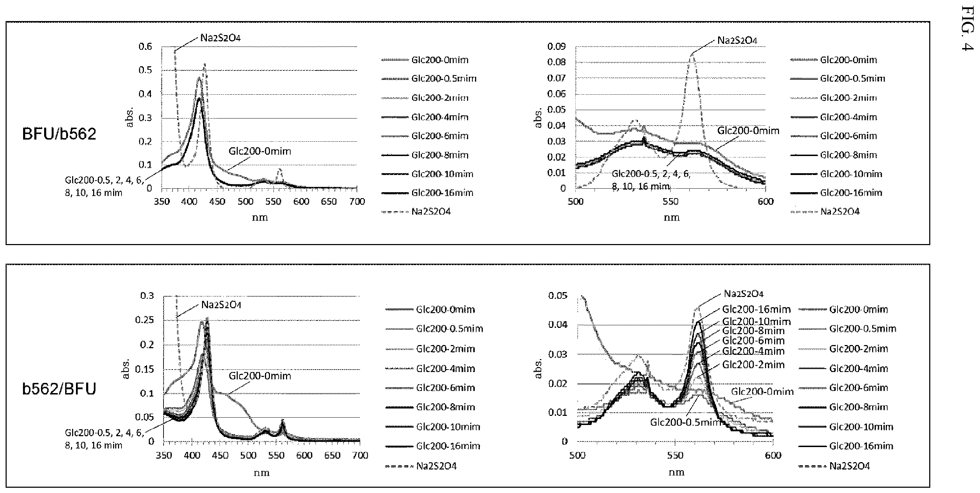

FIG. 4 illustrates the results of measuring the UV-VIS spectra of a fusion protein (BFU/b562) and a fusion protein at 350 to 700 nm. Top: BFU/b562, bottom: b562/BFU. In each of the top and the bottom, left figure: the results of measurement in the range of 350 to 700 nm, right figure: the results of measurement in the range of 500 to 600 nm.

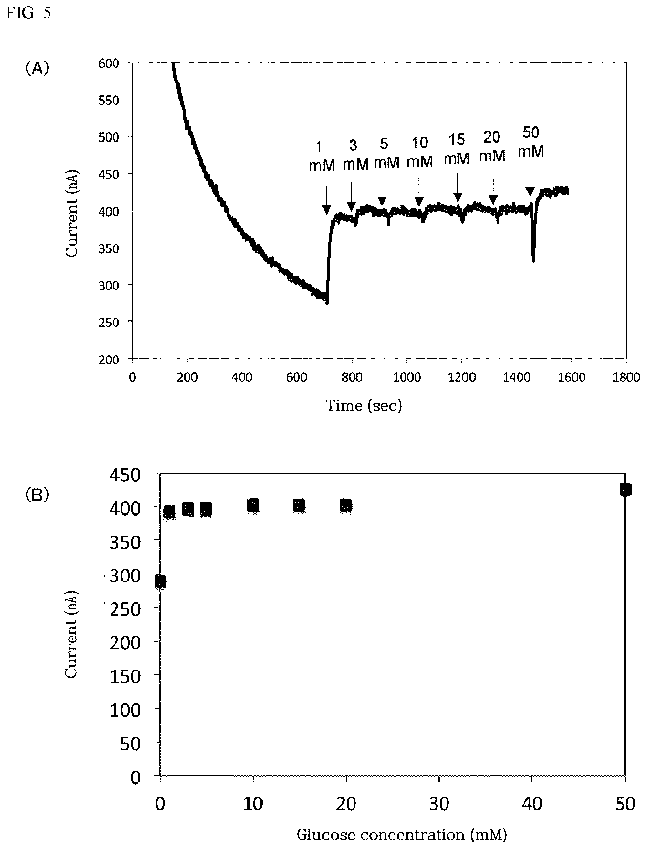

FIG. 5 depicts graphs illustrating the results of electrochemical measurement using an electrode having a fusion protein (b562/BFU) immobilized thereon.
   (A) illustrates the response results obtained when adding glucose at various concentrations. (B) illustrates a calibration curve.

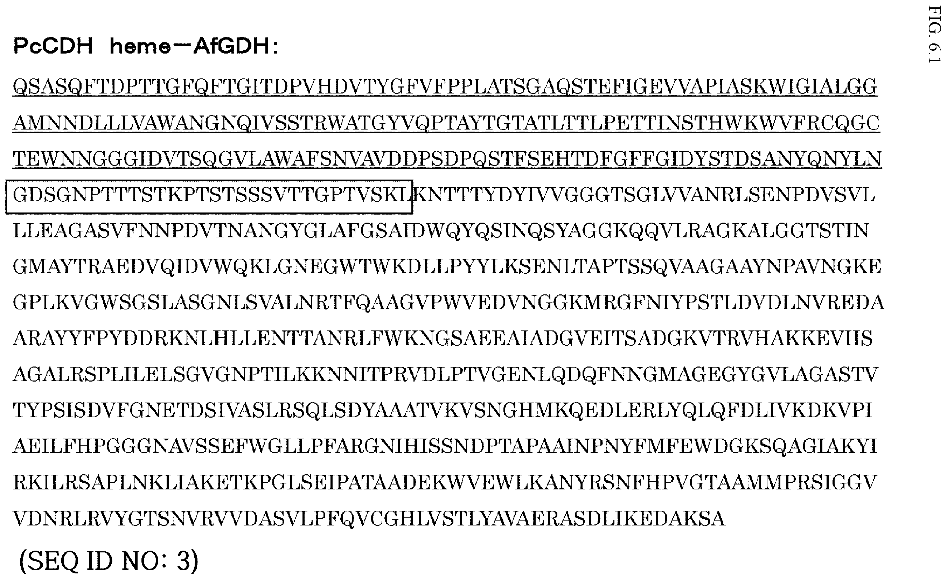

FIG. 6-1 illustrates the amino acid sequence (SEQ ID NO: 3) of a fusion protein (PcCDH heme-AfGDH) obtained by linking the cytochrome domain of fungal cellobiose dehydrogenase to the N-terminus of a fungus-derived FADGDH (AfGDH) via a linker sequence. The underlined portion indicates the sequence of the cytochrome domain, and the boxed sequence indicates the linker sequence. This sequence lacks the N-terminal signal sequence of the cytochrome domain, and the signal sequence of an α-factor derived from an expression vector may be added.

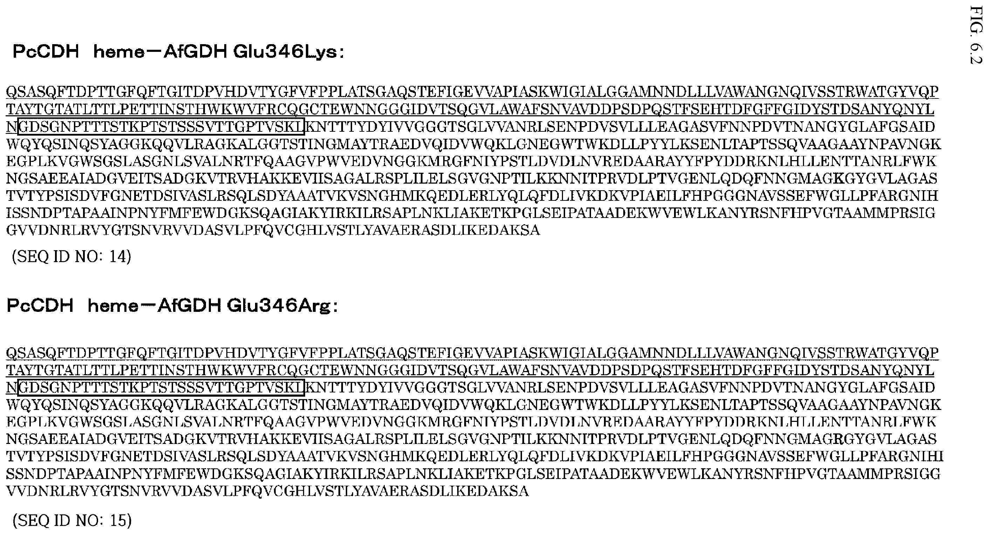

FIG. 6-2 illustrates the amino acid sequence (SEQ ID NOs: 14 and 15) of a fusion protein (PcCDH heme-AfGDHGlu346Lys or Arg) obtained by linking the cytochrome domain of fungal cellobiose dehydrogenase to the N-terminus of a fungus-derived FADGDH (AfGDHGlu346Lys or Arg) via a linker sequence. The underlined portion indicates the sequence of the cytochrome domain, and the boxed sequence indicates the linker sequence. The bold letter indicates mutation. This sequence lacks the N-terminal signal sequence of the cytochrome domain, and the signal sequence of an α-factor derived from an expression vector may be added.

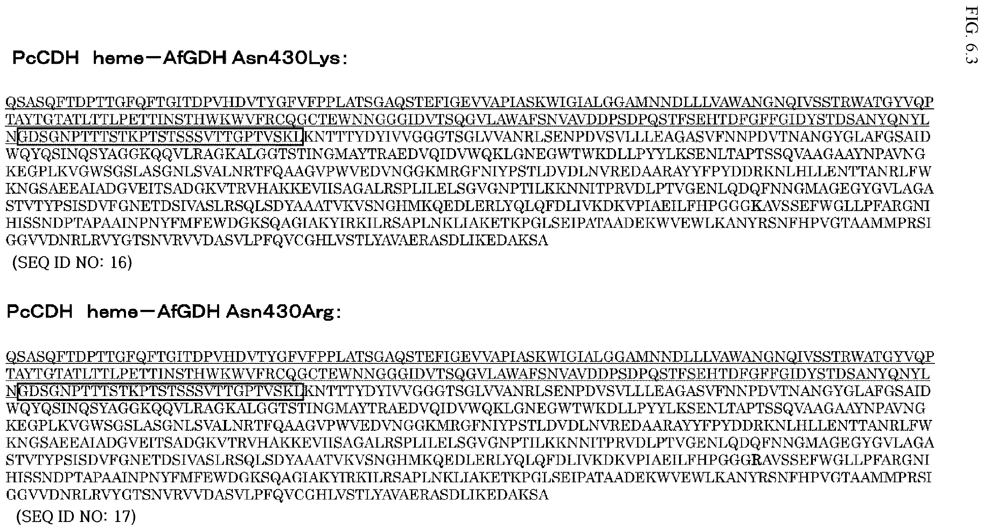

FIG. 6-3 illustrates the amino acid sequence (SEQ ID NOs: 16 and 17) of a fusion protein (PcCDH heme-AfGDHAsn430Lys or Arg) obtained by linking the cytochrome domain of fungal cellobiose dehydrogenase to the N-terminus of a fungus-derived FADGDH (AfGDHAsn430Lys or Arg) via a linker. The underlined portion illustrates the sequence of the cytochrome domain, and the boxed sequence indicates the linker sequence. The underlined portion indicates the sequence of the cytochrome domain, and the boxed sequence illustrates the linker sequence. The bold letter indicates mutation. This sequence lacks the N-terminal signal sequence of the cytochrome domain, and the signal sequence of an α-factor derived from an expression vector may be added.

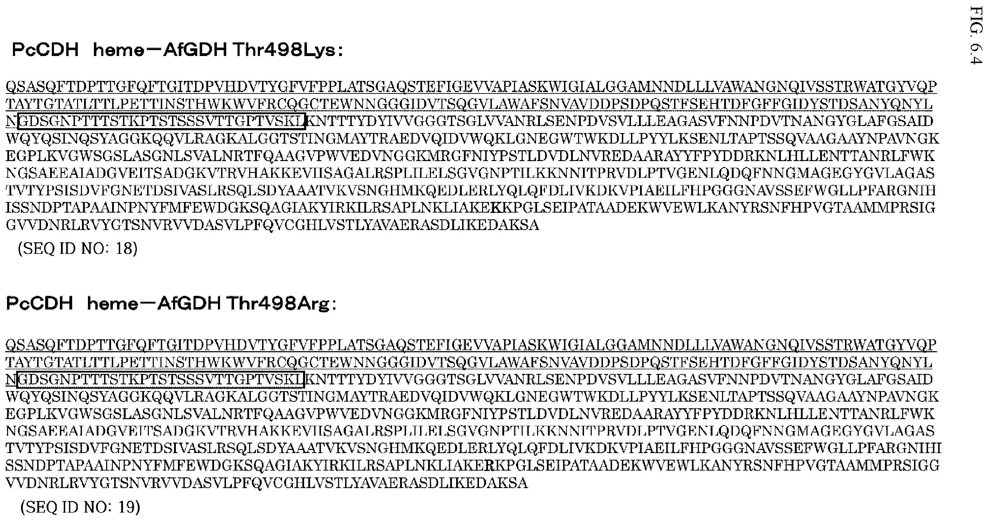

FIG. 6-4 illustrates the amino acid sequence (SEQ ID NOs: 18 and 19) of a fusion protein (PcCDH heme-AfGDHThr498Lys or Arg) obtained by linking the cytochrome domain of fungal cellobiose dehydrogenase to the N-terminus of a fungus-derived FADGDH (AfGDHThr498Lys or Arg) via a linker. The underlined portion indicates the sequence of the cytochrome domain, and the boxed sequence indicates the linker sequence. The bold letter indicates mutation. This sequence lacks the N-terminal signal sequence of the cytochrome domain, and the signal sequence of an α-factor derived from an expression vector may be added.

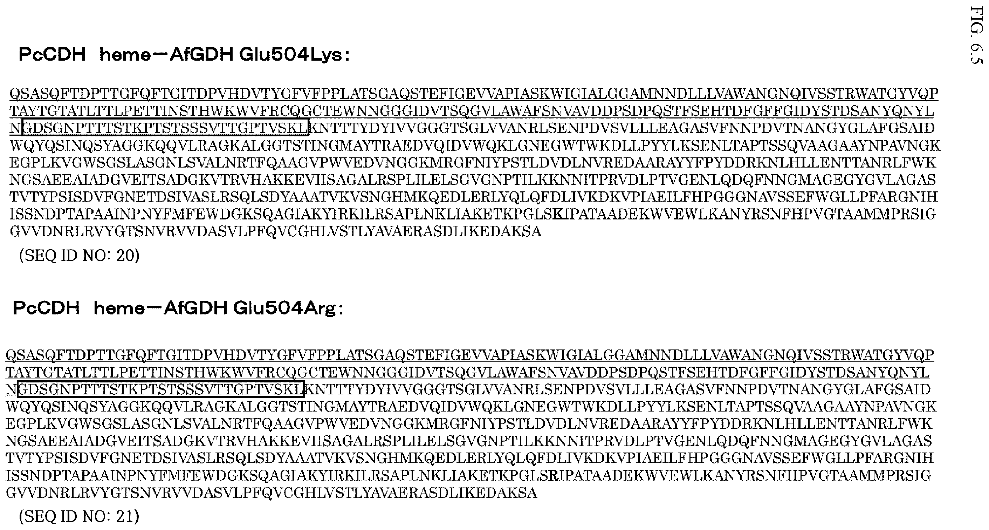

FIG. 6-5 illustrates the amino acid sequence (SEQ ID NOs: 20 and 21) of a fusion protein (PcCDH heme-AfGDHGlu504Lys or Arg) obtained by linking the cytochrome domain of fungal cellobiose dehydrogenase to the N-terminus of a fungus-derived FADGDH (AfGDHGlu504Lys or Arg) via a linker. The underlined portion indicates the sequence of the cytochrome domain, and the boxed sequence indicates the linker sequence. The bold letter indicates mutation. This sequence lacks the N-terminal signal sequence of the cytochrome domain, and the signal sequence of an α-factor derived from an expression vector may be added.

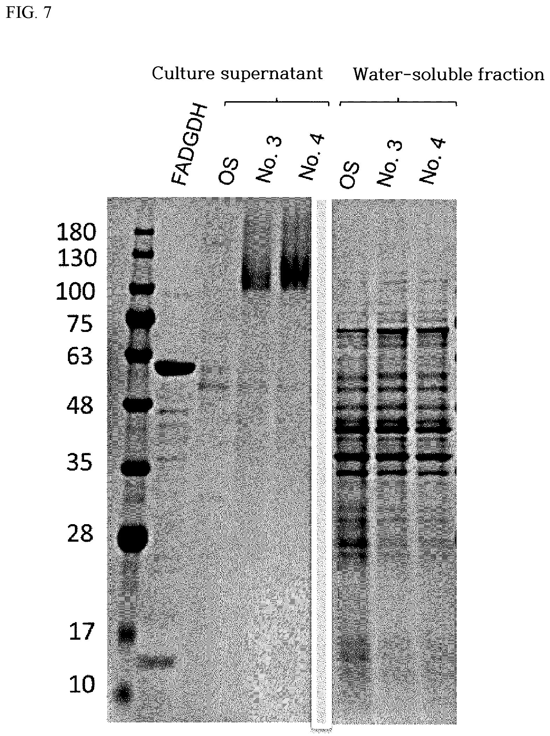

FIG. 7 is a photograph illustrating the results of SDS-PAGE analysis of the culture supernatant fraction of each of non-transformed host cells (OS), and transformants (sample No. 3 and No. 4) introduced with a gene encoding a fusion protein (PcCDH heme-AfGDH), and each water-soluble fraction obtained after dialyzing each of the supernatants.

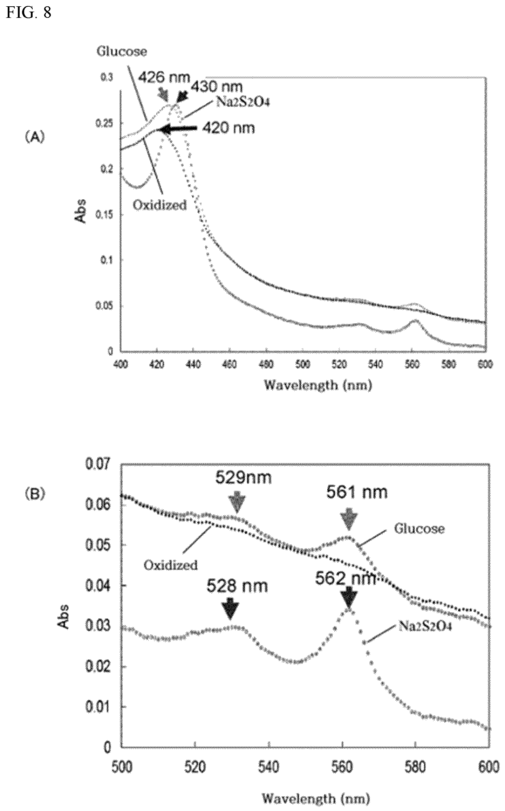

FIG. 8 illustrates the results of measuring the UV-VIS spectrum of a fusion protein (PcCDH heme-AfGDH) at 400 to 600 nm.
   (A) illustrates the results of measurement in the range of 400 to 600 nm, and (B) illustrates the results of measurement in the range of 500 to 600 nm.

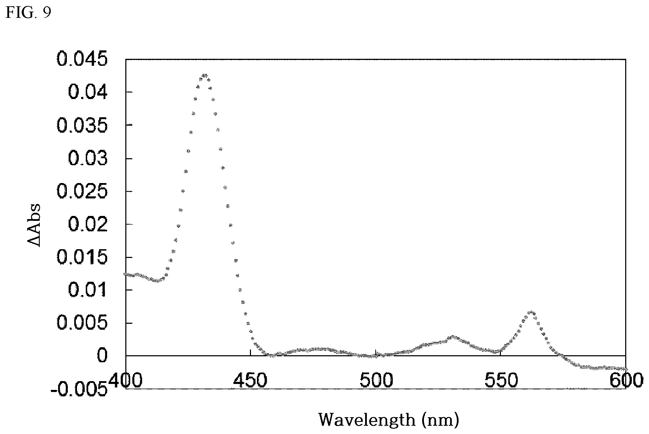

FIG. 9 illustrates the spectral difference in the UV-VIS spectral measurement (400 to 600 nm) of a fusion protein (PcCDH heme-AfGDH) between before and after addition of glucose.

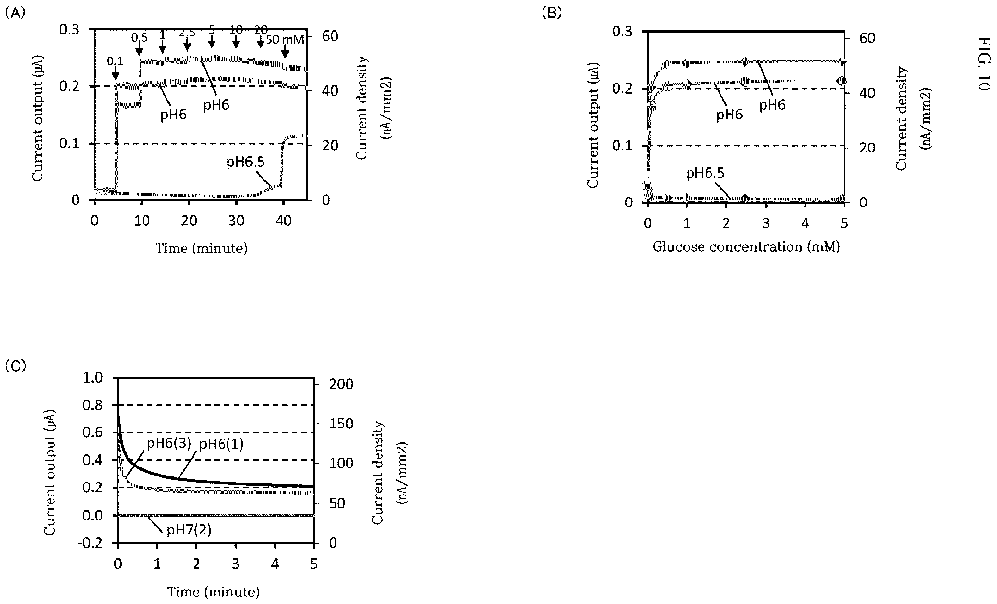

FIG. 10 depicts graphs illustrating the results of electrochemical measurement performed using electrodes having a fusion protein (PcCDH heme-AfGDH) immobilized thereon.
   (A) illustrates the response results obtained when adding glucose at various pHs, and (B) illustrates a calibration curve.
   (A) and (B) illustrate the results of electrochemical measurement at pH 6, performed using two separately fabricated electrodes. (C) illustrates the response results obtained when changing the pH of the reaction solution in the order of (1) pH 6, (2) pH 7, and (3) pH 6.
FIG. 11 is a photograph illustrating the results of SDS-PAGE of a wild-type AfGDH fusion protein and fusion proteins including AfGDH variants (E346K, N430K, T498K, and E504K), expressed in transformants.
FIG. 12 illustrates the results of measuring the UV-VIS spectra (400 to 600 nm) of a wild-type AfGDH fusion protein and fusion proteins including AfGDH variants (E346K, N430K, T498K, and E504K).
FIG. 13 depicts graphs illustrating the results of electrochemical measurement at pH 6, performed using a wild-type AfGDH fusion protein and fusion proteins including AfGDH variants (E346K, N430K, T498K, and E504K).

### MODE FOR INVENTION

### 1. Fusion protein

The fusion protein of the present disclosure is a fusion protein according to claim 1 comprising: a fungus-derived FADGDH or a variant thereof; and a cytochrome molecule linked to the N-terminus of the FADGDH or variant thereof.

Flavin adenine dinucleotide glucose dehydrogenase (FADGDH) is a glucose dehydrogenase that uses flavin adenine dinucleotide (FAD) as a coenzyme, and has an activity of catalyzing a reaction that produces gluconolactone by oxidizing glucose in the presence of an electron acceptor.

In the present disclosure, FADGDH is a fungus-derived FADGDH that is part of a fusion protein according to claim 1. A fungus-derived FADGDH include one derived from *Aspergillus* sp. and *Botryotinia* sp. Other examples of fungus-derived FADGDH not forming part of the invention include *Sclerotinia* sp., *Glomerella* sp., *Mucor* sp., *Parasitella* sp., *Rhizopus* sp., *Penicillium* sp., *Trichoderma* sp., *Colletotrichum* sp., *Talaromyces* sp., *Absidia* sp., *Actinomucor* sp., *Circinella* sp., *Aureobasidium* sp., *Kabatiella* sp., *Cladosporium* sp., *Fusicladium* sp., *Dumontinia* sp., *Ovulinia* sp., *Sclerotinia* sp., *Botrytis* sp., Ciborinia sp. Among them, an FADGDH derived from *Botryotinia* sp. may be very properly used. In particular, the fungus-derived FADGDH is preferably an FADGDH derived from *Aspergillus flavus* or *Botryotinia fuckeliana.* Other examples, not forming part of the invention, include *Mucor prainii, Mucor circinelloides, Mucor subtilissimus, Mucor guilliermondii, Mucor javanicus, Parasitella parasitica, Rhizopus microspores,* or the like.

Fungus-derived FADGDHs are known. Sequence information on the FADGDH derived from *Aspergillus flavus* is available from known databases. For example, the sequence information is available under XP_002372599.1 from the database of NCBI (National Center for Biotechnology Information). Furthermore, sequence information on the FADGDH derived from *Botryotinia fuckeliana* is disclosed in WO2012/001976. In addition, sequence information on the FADGDHs derived from *Mucor prainii, Mucor guilliermondii* and *Mucor hiemalis* NBRC6754 are disclosed in Japanese Patent Application Laid-Open Nos. 2014-18096, 2017-000137 and 2015-84676, respectively.

An FADGDH that may be used in the present disclosure is an FADGDH comprising or consisting of a protein represented by SEQ ID NO: 4 (FADGDH derived from *Aspergillus flavus*)and SEQ ID NO: 5 (FADGDH derived from *Botryotinia fuckeliana*))*.*

In addition, the FADGDH in the present disclosure also includes a variant thereof, as long as it retains the catalytic activity and is according to claim 1. The variant of the FADGDH may generally be represented by the following (I) or (II):
(I) a protein which consists of an amino acid sequence resulting from deletion, substitution, insertion or addition of one amino acid or a plurality of amino acids in the amino acid sequence of the FADGDH, and which has the catalytic activity; and
(II) a protein which consists of an amino acid sequence having a sequence identity of 85% or higher to the amino acid sequence of the FADGDH, and which has the catalytic activity.

The "plurality of amino acids" in (I) refers to about 2 to 120, preferably about 2 to 60, more preferably about 2 to 30 amino acids.

In addition, the "sequence identity" in (II) may be obtained according to a known sequence comparison method. For example, the sequence identity may be obtained by using BLAST (Basic Local Alignment Search Tool at the National Center for Biological Information), for example, as a default setting. The "sequence identity of 85% or higher" refers to a sequence identity of, for example, 85% or higher, preferably 90% or higher, more preferably 95% or higher, even more preferably 99% or higher.

Variants of the FADGDH include known FADGDH variants. These variants include variants of *Aspergillus flavus-*derived FADGDH, which are disclosed in WO2013/164477 and Japanese Patent Application Laid-Open Nos. 2016-208915, 2016-7193 and 2016-7192; variants of *Botryotinia fuckeliana*-derived FADGDH, which are disclosed in WO2012/001976. Other examples, not forming part of the invention, include variants of *Mucor prainii*-derived FADGDH, which are disclosed in WO2016/076364, Japanese Patent Application Laid-Open No. 2015-084676, WO2015/129475, WO2015/099112, WO2013/065770, WO2012/169512, WO2012/073987, WO2012/073986, and WO2010/140431; and variants of FADGDHs derived from other fungi, which are disclosed in WO2014/045912, WO2013/147206, WO2013/031664 and Japanese Patent Application Laid-Open Nos. 2016-208916, 2016-7191 and 2015-167506. According to an aspect, an FADGDH variant that may be used in the present disclosure may comprise or consist of a protein represented by an amino acid sequence having one or more mutations selected from the group consisting of Glu346Lys or Arg, Asn430Lys or Arg, Thr498Lys or Arg, and Glu504Lys or Arg in the amino acid sequence of *Aspergillus flavus* FADGDH (e.g., the amino acid sequence of SEQ ID NO: 4)..

The "cytochrome molecule" refers to a heme protein functioning as an electron carrier, and in particular, it refers to a protein molecule to which one or more heme irons are covalently or non-covalently bound. In addition, the "cytochrome molecule" in the present disclosure also includes a part of a protein that retains electron transfer function, called an electron transfer subnit, a heme-containing domain, or a quinohemoprotein subunit or domain in oxidoreductase. Hereinafter, a part of the protein that retains electron transfer function is sometimes referred to as a "cytochrome domain". Examples of the oxidoreductase include, but are not limited to, cellobiose dehydrogenase, ethanol dehydrogenase, oligosaccharide dehydrogenase, and the like.

Various kinds of cytochrome molecules have been isolated and identified from various organisms. Preferably, cytochrome b is used.

Cytochrome molecules that are used in the present disclosure include cytochrome molecules derived from bacteria. The bacteria that are used are *Escherichia* sp. Other examples of bacteria, not forming part of the invention include *Pseudomonas* sp., *Acinetobacter* sp., *Klebsiella* sp., *Salmonella* sp., *Yersinia* sp., *Pasteurella* sp., *Rhizobium* sp., *Comamonas* sp., and the like. Preferably, a cytochrome molecule derived from *Escherichia coli* is used.Other examples, not forming part of the invention, include *Pseudomonas stutzeri, Acinetobacter calcoaceticus, Klebsiella pneumoniae, Salmonella typhi, Salmonella typhinulium,* and *Yersinia pestis.* In particular, cytochrome b562 derived from *Escherichia coli* is used. Cytochrome molecules derived from bacteria are known. Sequence information on cytochrome b562 is available from known database. For example, the sequence information is available under WP_057688639.1 from the database of NCBI. In the present disclosure, these known cytochrome molecules derived from bacteria may also be used.

In addition, examples of cytochrome molecules that may be used in the present disclosure include the cytochrome domains of oxidoreductases derived from fungi or bacteria. The fungi include, filamentous fungi, more particularly *Phanerochaete* sp. Other examples, not forming part of the invention, include *Grifola* sp., *Pycnoporus* sp., *Trametes* sp., *Humicola* sp., *Thielavia* sp., *Irpex* sp., *Schizophyllum* sp., *Coniophora* sp., and *Sclerotium* sp. Examples of the bacteria include the above-described bacteria. Preferably, the cytochrome molecule of cellobiose dehydrogenase derived from filamentous fungi, more particularly *Phanerochaete* sp. is used. Other examples, not forming part of the present invention, include cytochrome molecule of cellobiose dehydrogenase of *Grifola* sp., *Pycnoporus* sp., *Trametes* sp., *Humicola* sp., or *Thielavia* sp. In particular, the cytochrome molecule is preferably the cytochrome domain of cellobiose dehydrogenase from *Phanerochaete chrysosporium.* Other examples, not forming part of the invention, include *Grifola frondosa, Pycnoporus cinnabarinus, Trametes versicolor, Humicola insolens,* and *Thielavia heterothallica.*

The cytochrome domains of oxidoreductases from fungi or bacteria are known. Sequence information on cellobiose dehydrogenase from *Phanerochaete chrysosporium* is available from known databases. For example, the sequence information is available under GenBank: AAB61455.1, GenBank: AAC49277.1, or the like. In the present disclosure, these known cytochrome domains of oxidoreductases from fungi or bacteria may also be used.

According to an aspect, a cytochrome molecule that may be used in the present disclosure may comprise or consist of a protein represented by the amino acid sequence of SEQ ID NO: 8 (the amino acid sequence of cytochrome b562 from *E. coli)* or SEQ ID NO: 9 (the amino acid sequence of the cytochrome domain (classified as cytochrome b) of cellobiose dehydrogenase from *Phanerochaete chrysosporium*)*.*

In addition, the cytochrome molecule in the present disclosure also includes a variant thereof, as long as it retains electron carrier function and is according to claim 1. A variant of the cytochrome molecule may be represented by the following (i) or (ii):
(i) a protein which consists of an amino acid sequence resulting from deletion, substitution, insertion or addition of one amino acid or a plurality of amino acids in the amino acid sequence of the cytochrome molecule, and which has the above-described function; and
(ii) a protein which consists of an amino acid sequence having a sequence identity of 85% or higher to the amino acid sequence of the cytochrome molecule, and which has the above-described function.

The "plurality of amino acids" in (i) refers to 2 to 40, preferably 2 to 20, more preferably 2 to 10 amino acids.

In addition, the "sequence identity" in (ii) is as defined above. The "sequence identity of 85% or higher" refers to a sequence identity of, for example, 85% or higher, preferably 90% or higher, more preferably 95% or higher, even more preferably 99% or higher.

In the present disclosure, regarding the "linking", the FADGDH or variant thereof and the cytochrome molecule may be bound directly to each other or may be bound to each other via a linker sequence.

As the linker sequence, a peptide linker may be used, and the number and type of amino acids thereof are not particularly limited. For example, a linker sequence having about 1 tc 100 amino acids, preferably about 5 to 50 amino acids, or particularly preferably about 10 to 30 amino acids may be used. As the linker sequence, any peptide linker known in the related art may be used, or the loop sequence of oxidoreductase may be very preferably used. For example, a polypeptide comprising an amino acid sequence (e.g., the amino acid sequence represented by SEQ ID NO: 12) selected from the loop sequence of cellobiose dehydrogenase from *Phanerochaete chrysosporium* may be used as the peptide linker.

In one embodiment of the fusion protein of the present disclosure, the fusion protein comprises an FADGDH from *Botryotinia fuckeliana* or a variant thereof and cytochrome b562 from *E. coli,* and has a structure in which the cytochrome b562 and the FADGDH are sequentially linked at the N terminus via a linker. One embodiment of this fusion protein may have a structure illustrated in at least one of FIGS. 2-1 and 2-2.

In other embodiments of the fusion protein of the present disclosure, the fusion protein comprises an FADGDH from *Aspergillus flavus* or a variant thereof and a cytochrome domain of cellobiose dehydrogenase from *Phanerochaete chrysosporium,* and has a structure in which the cytochrome domain and the FADGDH are sequentially linked at the N terminus via a linker. One embodiment of this fusion protein may have a structure illustrated in at least one of FIGS. 6-1 to 6-5.

The fusion protein of the present disclosure is a protein having glucose dehydrogenase activity in the moiety of the FADGDH or the variant thereof and having electron transfer function in the cytochrome molecule moiety. The moiety of the FADGDH or the variant thereof recognizes glucose and catalyzes the redox reaction thereof, and electrons generated by the redox reaction are transferred to the heme of the cytochrome molecule by internal electron transfer (IET), and then transferred directly to an external electron acceptor.

According to an aspect, the fusion protein of the present disclosure comprises or consists of (a) an amino acid sequence set forth in at least one of SEQ ID NOs: 2, 3 and 13 to 21.

In addition, the fusion protein in the present disclosure also includes a variant thereof, as long as it retains the glucose dehydrogenase activity and electron transfer function. The variant may generally be represented by the following (b):
(b) a protein which has a sequence identity of 85% or higher to the amino acid sequence of (a) and which has glucose dehydrogenase activity and electron transfer function.

The "sequence identity" in (b) is as defined above. The "sequence identity of 85% or higher" refers to a sequence identity of, for example, 85% or higher, preferably 90% or higher, more preferably 95% or higher, even more preferably 99% or higher.

Preferably, if the amino acid residues at positions 538, 622, 690 and 696 in SEQ ID NOs: 14 to 21 are Lys or Arg, any of the amino acid residues (Lys or Arg) at these positions does not change.

The fusion protein of the present disclosure may have, at the N-terminus or C-terminus thereof, an additional sequence such as a tag sequence (e.g., a histidine tag sequence, etc.) for purification or a signal sequence (e.g., an α-factor secretion signal sequence) for secretion, if necessary.

### 2. Method for production of fusion protein

Production of the fusion protein may be performed based on a general gene recombination method known to those skilled in the art (Green, M. R. and Sambrook, J., 2012, Molecular Cloning: A Laboratory Manual Fourth Ed., Cold Spring Harbor Laboratory Press, Cold Spring Harbor, New York).

That is, the fusion protein of the present disclosure may be obtained by inserting a nucleic acid encoding the fusion protein into a suitable expression vector, introducing and expressing the nucleic acid in a suitable host cell, and isolating and purifying the expressed fusion protein. As used herein, the "nucleic acid" refers to DNA, RNA, a DNA/RNA hybrid, and the form thereof is not particularly limited as long as it encodes a desired protein.

The nucleic acid encoding the fusion protein of the present disclosure may be produced by linking a nucleic acid encoding the FADGDH or the variant thereof in-frame to a nucleic acid encoding the cytochrome molecule, if necessary, through a nucleic acid encoding a linker.

The nucleic acid encoding each of the proteins may be prepared based on a general method known in the related art. For example, from a cDNA library or genome library prepared from cells containing each protein, PCR may be performed using primers designed and synthesized based on the nucleotide sequences of nucleic acids encoding each protein, whereby the desired nucleic acid may be amplified from the library and isolated. Since the primers may have restriction enzyme recognition sequences, the restriction enzyme recognition sequences may be provided in the nucleic acid isolated thereby, and inter-ligation and/or ligation to a vector may be performed using the sequences. Alternatively, the nucleic acid encoding the fusion protein of the present disclosure may also be a chemically synthesized nucleic acid having a desired sequence. In addition, if desired mutation is introduced into each nucleic acid, a site-specific mutation introduction method using a PCR method may be used.

The obtained nucleic acids encoding each protein are linked into a suitable expression vector in a desired order by restriction enzymes and DNA ligases. The nucleic acid encoding the fusion protein of the present disclosure is operably linked to expression control sequences comprising a promoter. The "operably linked" means that the inserted nucleic acid is expressed under the control of a promoter in a host cell.

The expression vector may be any one capable of replicating or surviving in a host cell, and examples thereof include, but are not particularly limited to, plasmid vectors, phage vectors, cosmid vectors, phagemid vectors, viral vectors, etc. The promoter contained in the expression vector may be any promoter as long as it is appropriate for the host cell to be used. For example, if the host cell is *E. coli,* lac promoter, Trp promoter, PL promoter, PR promoter or the like may be used, and if the host cell is yeast, AOX1 promoter, TEF1 promoter, ADE2 promoter, CYC1 promoter, GAL-L1 promoter, or the like may be used. (However, the present disclosure is not limited thereto.)

The expression vector may additionally contain a selectable marker (e.g., an antibiotic resistance gene, an auxotrophic gene, etc.) for selecting transformants, a replication origin enabling replication in the host, an enhancer, a terminator region, a poly A addition signal, a tag sequence for purification, a signal sequence for secretion, or the like.

The host cell into which the expression vector is to be introduced may be any host cell that may express the fusion protein of the present disclosure. As the host cell, bacteria, fungi, insect cells, animal cells (e.g., mammalian cells), plant cells, or the like may be used. Preferably, the host cell is of the same type as the cell from which the cytochrome molecule is derived. If the cytochrome molecule is derived from bacteria, bacteria may preferably be used as the host cell, and if the cytochrome molecule is derived from fungi, fungi may preferably be used as the host cell. (However, the levels of the genus or species of the host cell and the cell from which the cytochrome molecule is derived may be the same or different.) For example, if the host cell is bacteria, *E*. *coli* may be used, and if the host cell is fungi, yeasts, such as *Pichia, Saccharomyces* or *Schizosaccharomyces,* or fungi, such as *Aspergillus* or *Trichoderma,* may be used.

Transformation of the host cell with the expression vector may be performed by a known method, such as a calcium phosphate method, an electroporation method, a lipofection method, a particle gun method, or a PEG method.

The transformed host cell may be cultured according to a conventional method. If the fusion protein of the present disclosure has a signal sequence for secretion and is secreted extracellularly after production by expression, a desired peptide may be recovered from the cell culture. Alternatively, a desired peptide accumulated in the cell may also be recovered. In this case, the cell is physically or chemically disrupted (e.g., by sonication, homogenization, lysis, etc.), and a desired peptide is recovered using protein purification technology.

The recovered fusion protein of the present disclosure may be purified by a conventional method, for example, chromatography, such as gel filtration chromatography, ion exchange column chromatography, affinity chromatography, reverse phase chromatography, or HPLC, ammonium sulfate fractionation, ultrafiltration, or immunoadsorption.

According to an aspect, the nucleic acid encoding the protein that may be used in production of the fusion protein of the present disclosure comprises or consists of (A) a polynucleotide represented by a nucleotide sequence represented by at least one of SEQ ID NOs: 10, 11 and 26 to 34. The nucleotide sequences represented by SEQ ID NOs: 10, 11, 26, 27, 28, 29, 30, 31, 32, 33 and 34 encode the amino acid sequences represented by SEQ ID NOs: 2, 3, 13, 14, 15, 16, 17, 18, 19, 20 and 21, respectively.

In addition, the nucleic acid protein of the present disclosure also includes a variant thereof, as long as it encodes a protein having glucose dehydrogenase activity and electron transfer function. A variant of the nucleic acid may generally be represented by at least one of the following (B) to (D):
(B) a polynucleotide which consists of a nucleotide sequence resulting from deletion, substitution, insertion or addition of one nucleotide or a plurality of nucleotides in the nucleotide sequence of (A), and which has glucose dehydrogenase activity and electron transfer function;
(C) a polynucleotide which consists of a nucleotide sequence having a sequence identity of 80% or higher to the nucleotide sequence of (A), and which has glucose dehydrogenase activity and electron transfer function; and
(D) a polynucleotide which consists of a nucleotide sequence that hybridizes completely or at least 80% with a sequence complementary to the nucleotide sequence of (A) under stringent conditions, and which has glucose dehydrogenase activity and electron transfer function.

The "plurality of nucleotides" in (B) refers to 2 to 400, preferably 2 to 200, more preferably 2 to 100 nucleotides.

In addition, the "sequence identity" in (C) is as defined above. The "sequence identity of 80% or higher" refers to a sequence identity of, for example, 85% or higher, preferably 90% or higher, more preferably 95% or higher, even more preferably 99% or higher.

In addition, the "stringent conditions" in (D) refers to conditions under which a specific hybrid is formed whereas a non-specific hybrid is not formed. For example, the "stringent conditions" refers to conditions under which hybridization is performed in a solution containing 2 to 6×SSC (the composition of 1×SSC: 0.15 M NaCl, 0.015 M sodium citrate, and pH 7.0) and 0.1 to 0.5% SDS at 42 to 55°C, and washing is performed in a solution containing 0.1 to 0.2×SSC and 0.1 to 0.5% SDS at 55 to 65°C. The "80% or higher" refers to 85% or higher, preferably 90% or higher, more preferably 95% or higher, even more preferably 99% or higher, of the entire nucleotide sequence.

Preferably, if the amino acid residues at positions 538, 622, 690 and 696 in SEQ ID NOs: 14 to 21 are Lys or Arg, any of the amino acid resides (Lys or Arg) at these positions in the amino acid sequence encoded by the variant of the nucleic acid does not change.

### 3. Enzyme electrode

The present disclosure also relates to an enzyme electrode having the fusion protein of the present disclosure immobilized thereon. The "enzyme electrode" refers to an electrode having an enzyme immobilized on the surface of an electrode, such as a gold electrode, a platinum electrode or a carbon electrode. The enzyme electrode may be widely used as a biosensor that uses the reaction specificity of an enzyme to specifically detect a variety of biologically active substances. The fusion protein immobilized on the surface of the enzyme electrode of the present disclosure recognizes the presence of an analyte (e.g., glucose) in a sample, and catalyzes the redox reaction of the analyte. The electrodes resulting from the redox reaction are transferred to the heme of the cytochrome molecule by internal electron transfer, and then transferred directly to the external electron acceptor electrode.

The enzyme electrode of the present disclosure may be fabricated by immobilizing the fusion protein of the present disclosure on the surface of an electrode. The method of immobilizing the fusion protein on the electrode surface may use one of a method using a crosslinking reagent, a method of encapsulating the fusion protein into a polymer matrix, a method of coating the fusion protein with a dialysis membrane, a method of immobilizing the fusion protein in a polymer, such as a photo-crosslinking polymer, an electrically conductive polymer or a redox polymer, and combinations thereof. Typically, fabrication of the enzyme electrode may be performed by immobilizing the fusion protein of the present disclosure on a carbon electrode using glutaraldehyde, and then blocking the glutaraldehyde by treatment with a reagent having an amine group. The obtained enzyme electrode may be used as a working electrode of a glucose sensor.

### 4. Glucose sensor

The "glucose sensor" in the present disclosure refers to a measurement system for electrochemically measuring the concentration of glucose in a sample. Examples of the sample include blood samples, blood dialysis samples, blood, or blood or interstitial fluids.

The glucose sensor of the present disclosure may have a two-electrode structure comprising the enzyme electrode of the present disclosure as a working electrode and a counter electrode (e.g., a platinum electrode), or may have a three-electrode structure further comprising a reference electrode (e.g., an Ag/AgCl electrode). The working electrode may be one working electrode and may comprise a plurality of lines. The glucose sensor may further comprise a power source for applying a voltage to the working electrode, an ampere meter, a recorder, and a display unit. The configuration of the glucose sensor of the present disclosure is not particularly limited, and a known configuration (e.g., WO2005/030807, WO2005/023111, etc.) may be adopted. In addition, the glucose sensor of the present disclosure may also be referred to as being disposable.

### 5. Measurement of glucose concentration

Measurement of the glucose concentration using the glucose sensor of the present disclosure may be performed as follows. That is, as a working electrode, an enzyme electrode having the fusion protein of the present disclosure immobilized thereon is used, and as a counter electrode, a platinum electrode, for example, is used, and as a reference electrode, an Ag/AgCl electrode, for example, is used. After a constant voltage is applied to the working electrode and the current reaches a steady state, a sample is brought into contact with the enzyme electrode, and then electrons generated by the oxidation of glucose in the sample are measured using an increase in the current as an index. The glucose concentration in the sample may be calculated according to the calibration curve of the current increase, prepared using a standard-concentration glucose solution.

### Examples

Hereinafter, the present disclosure will be described in detail with reference to examples, but the present disclosure is not limited to these examples. The scope of protection is determined by the claims.

### Example 1: Fusion Protein Comprising Fungus-Derived FADGDH and Ethanol Dehydrogenase-Derived Cytochrome Molecule

### (1) Construction of Fusion Protein

An examination of whether or not an enzyme having direct electron transfer (DET) ability could be constructed by linking a cytochrome molecule to a fungus-derived FADGDH was made. First, an examination of whether or not an enzyme having DET ability could be constructed by linking a cytochrome molecule at the gene level to the C-terminus of a fungus-derived FADGDH according to a previously reported configuration was made.

In addition, an examination of whether or not an enzyme having DET ability could be constructed by linking a cytochrome molecule at the gene level to the N-terminus of a fungus-derived FADGDH was made according to a completely new idea.

FIG. 1 illustrates the amino acid sequence of a fusion protein which is recombinantly produced based on a gene constructed by linking *E*. coli-derived cytochrome b562 at the gene level to the C-terminus of a fungus-derived FADGDH in this Example. In addition, the "fungus-derived FADGDH" used in this Example is a variant (BFU) of *Botryotinia fuckeliana*-derived glucose dehydrogenase (BFUGDH) (WO2012/001976).

It was aimed at constructing a fusion protein (BFU/b562) in which electron transfer from the FAD of GDH to the heme occurs, by linking cytochrome b562 to the C-terminus of the FADGDH via the loop sequence of quinohemoprotein ethanol dehydrogenase (QHEDH) from *Comamonas testosteroni* as a linker sequence.

In addition, FIG. 2-1 illustrates the amino acid sequence of a fusion protein which is recombinantly produced based on a gene constructed by linking *E. coli-*derived cytochrome b562 at the gene level to the N-terminus of a fungus-derived FADGDH in this Example.

It was aimed at constructing a fusion protein (b562/BFU) in which electron transfer from the FAD of GDH to the heme occurs, by linking cytochrome b562 to the N-terminus of the FADGDH via the loop sequence of cellobiose dehydrogenase from *Phanerochaete chrysosporium* as a linker sequence.

Genes encoding each fusion protein designed as described above were constructed, and each of the genes was inserted into the expression vector pET30c, thereby constructing fusion protein expression vectors. An *E. coli* BL21(DE3) strain was transformed with each of the obtained expression vectors. As a control, transformation was performed using an expression vector comprising the FADGDH(Bfu)-encoding gene that does not comprise *E. coli-*derived cytochrome b562.

### (2) Recovery of Fusion Protein and Evaluation of Enzymatic Activity

### 2-1. Recovery Method

*E. coli* transformed with each expression vector was precultured, and 1% was inoculated into 60 mL of a Km-containing autoinduction LB liquid medium in a 300-mL baffled flask, and then shake-cultured at 125 rpm at 20°C for 28 hours (two cultures each).

50 mL of the cell culture was collected, and 1 g of the cells were added to and suspended to 5 mL of BugBuster protein extraction reagent and incubated at room temperature for 20 minutes while gently shaking.

After the insoluble fraction was removed by centrifugation at 15 Krpm at 4°C for 20 minutes, the obtained supernatant was dialyzed using 20 mM sodium phosphate buffer/0.5 M NaCl/20 mM imidazole (pH7.0) at 4°C.

After dialysis, the precipitate was removed by centrifugation (at 15 Krpm at 4°C for 20 minutes), and the supernatant was applied to an open column containing 1 mL Ni-NTA gel. After application, washing was performed with 10 ml of buffer A, and stepwise elution (5 mL each) was performed with (buffer A): (buffer B) = 90.5:9.5 to (buffer A): (buffer B) =75:25.

The supernatant and non-adsorbed fractions were placed in a microdialyzer (EasySep) and dialyzed against 20 mM potassium phosphate buffer (pH 6.5) at 4°C.

In addition, the 9.5% and 25% eluates were placed in a 3.5-kDa cutoff dialysis membrane and dialyzed against 20 mM potassium phosphate buffer (pH6.5) at 4°C.

The compositions of the buffer A and buffer B used are as follows.
Buffer A: 20 mM sodium phosphate buffer/0.5 MNaCl/20 mM imidazole (pH 7.0)
Buffer B: 20 mM sodium phosphate buffer/0.5 M NaCl/500 mM imidazole (pH 7.0)

After dialysis, the 25% eluate was concentrated with a 10-kDa cutoff ultrafiltration membrane to obtain a purified sample.

A sample for SDS-PAGE was prepared by mixing the eluate (5 µL), loading buffer (5 µL), mercaptoethanol (1 µL) and H₂O (9 µL) and heating the mixture at 95°C for 10 minutes, and 5 µL of the sample was applied.

### 2-2. Method of the evaluation of enzymatic activity

The GDH enzyme activity of the obtained fusion protein was measured using 0.06 mM DCIP and 0.6 mM MPMS in 20 mM potassium phosphate buffer (pH 6.5). As a substrate, 40 mM glucose (final concentration) was used. The activity of the enzyme that oxidizes 1 µmol of glucose for 1 minute was defined as 1 unit.

### 2-3. Results and discussion

After culture, the obtained transformants expressing BFU/b562 and the wet cells of the transformants expressing b562/BFU had a reddish color. After each of the wet cells was lysed, the red color of the lysed supernatant was very small, while the precipitate had a reddish color. In addition, the red color of the lysed supernatant was slightly strong in BFU/b562.

FIG. 3 illustrates the results of SDS-PAGE analysis. In SDS-PAGE, it could be confirmed that the fusion proteins (BFU/b562 and b562/BFU) were all expressed and produced as planned (a band around 75 kD). It was confirmed that BFU/b562 was partially cleaved (a band around 15 kD), the band was thick, indicating that the proportion of the cleavage was very small. In addition, in b562/BFU, the band of Bfu alone could hardly be seen, indicating that the cleavage of the protein hardly occurred.

Tables 1-1 and 1-2 below represent the results of measuring the production of each of BFU/b562 and b562/BFU and the activity of each enzyme.

The recovery rate of each fusion protein was 113.5% for BFU/b562 and 107.2% for b562/BFU, indicating that each fusion protein was almost all recovered.

In addition, the recovery of activity for the total activity was 96.4% for BFU/b562 and 72.5% for b562/BFU. Since the enzyme activity was low in some cases, it is suggested that adsorption or the like occurred during measurement or deactivation occurred in the purification step.

**[Table 1-1]**

| Glc 40mM PMS/DCIP | Amoun t (ml) | Prot ein (mg /ml) | Total prote in (mg) | Protein yield (%) | | U/ml | Total U | Total U% | | U/mg | |
|---|---|---|---|---|---|---|---|---|---|---|---|
| BFU/b562 | 8.0 | 4.61 | 36.9 | 100 | - | 121.7 | 973.6 | 100 | - | 27.09 | 1.00 |
| Through | 11.0 | 3.24 | 35.7 | 96.7 | 96.7 | 21.0 | 231.1 | 23.7 | 23.7 | 6.58 | 0.24 |
| 9.5% | 5.0 | 0.90 | 4.5 | 12.2 | 16.8 | 118.4 | 592.2 | 60.8 | 72.7 | 134.95 | 4.98 |
| 25% | 5.0 | 0.33 | 1.7 | 4.6 | | 23.2 | 115.7 | 11.9 | | 70.42 | 2.60 |
| Conc 25% | 4.51 | 4.51 | 1.1 | - | - | 823.9 | 206.0 | - | - | 189.95 | - |

**[Table 1-2]**

| Glc40mM PMS/DCIP | Amount (ml) | Prote in (mg /ml) | Total prote in (mg) | Protein yield (%) | | U/ml | Total U | Total U% | | U/mg | |
|---|---|---|---|---|---|---|---|---|---|---|---|
| b562/BFU | 8.0 | 3.84 | 30.7 | 100 | - | 54.3 | 434.0 | 100 | - | 14.99 | 1.00 |
| Through | 11.0 | 2.75 | 30.3 | 98. 7 | 98.7 | 4.1 | 44.9 | 10.3 | 10.3 | 1.60 | 0.11 |
| 9.5% | 5.0 | 0.44 | 2.2 | 7.2 | 8.5 | 49.0 | 244.8 | 56.4 | 52.2 | 118.18 | 7.89 |
| 25% | 5.0 | 0.07 | 0.4 | 1.3 | | 5.1 | 25.3 | 5.8 | | 71. 87 | 4.79 |
| Conc 25% | 0.25 | 6.14 | 1.5 | - | - | 1100. 3 | 275.1 | - | - | 184.61 | - |

### (3) Measurement of UV-VIS spectrum

### 3-1. Method

Measurement of the spectrum of reduced heme was performed according to a conventional known method. The 25% elution fraction concentrate of each fusion protein was diluted 5 times to obtain a sample, and the absorption spectrum at 350 to 700 nm was measured. That is, glucose (final concentration: 200 mM) was added to the sample (0.9 µg fusion protein/L) in 20 mM potassium phosphate buffer (pH 6.5) to a total volume of 200 µL, and after 0, 0.5, 2, 4, 6, 8, 10 and 16 minutes, spectral measurement was performed. In addition, a reducing agent (final concentration: 5 mM sodium dithionite) was added to the sample (0.9 µg fusion protein/L) to a final volume of 200 µL, and spectral measurement upon complete reduction was performed

### 3-2. Results

FIG. 4 illustrates the results of measurement of the UV-VIS spectrum. It could be confirmed that the fusion proteins all exhibited a reduction peak by the addition of the reducing agent, and thus contained heme having a function.

However, for BFU/b562, the reduction peak of heme could not be found even when glucose was added (FIG. 4, top). This result indicates that, when the cytochrome molecule was linked to the C-terminus of the fungus-derived FADGDH, electron transfer from FAD to heme did not occur. That is, for this fusion protein, the cytochrome molecule having DET ability was not reduced by addition of glucose. That is, it has become clear that, in the previously reported configuration, DET ability cannot be imparted to the fungus-derived FADGDH.

In addition, for b562/BFU, the reduction peak of heme by the addition of glucose could be found (FIG. 4, bottom). Thus, it was shown that, when heme was linked to the N-terminus of the fungus-derived FADGDH, electron transfer from FAD to the heme became possible.

As described above, the DET ability could be imparted to the fungus-derived FADGDH by linking the cytochrome molecule to the N-terminus of the FADGDH protein.

### (4) Electrochemical evaluation of DET ability

### 4-1. Method

The DET ability of b562/BFU was evaluated by the following method.

A 4% mesoporous carbon dispersion (10 µL) prepared by mixing mesoporous carbon (manufactured by Toyo Carbon Co., Ltd.) with acetone was dried to obtain mesoporous carbon particles. The mesoporous carbon particles and 8 µL of b562/BFU (4.9 mg/ml) were added to 1 µL of 100 mM potassium phosphate buffer (pH 7.0) and incubated at 25°C for 1 hour. 1 µL of 20% sucrose was added thereto to obtain ink, and 7 µL of the ink was dropped onto an Au electrode (7 mm²) and crosslinked (1 hour under a 25% glutaraldehyde atmosphere). The obtained material was blocked and washed with Tris buffer, and then stored in 100 mM potassium phosphate buffer (pH 7.0) until use.

Measurement was performed at 37°C in 100 mM potassium phosphate buffer (pH 7.0) using an electrode immobilized with a b562/BFU-immobilized electrode as a working electrode, a platinum electrode as a counter electrode, and an Ag/AgCl electrode as a reference electrode. A voltage of +0.4 V vs. Ag/AgCl was applied, and the increase in the current value by the addition of glucose was measured.

### 4-2. Results

FIG. 5 illustrates the response results of the b562/BFU-immobilized electrode. The rapid increase in the current value by the addition of 1 mM glucose was observed, indicating that b562/BFU had DET ability (FIG. 5(A)). In contrast, in the electrode fabricated in the same shape using the BFU alone, this response could not be seen.

These results clearly show that the DET ability could be imparted to the fungus-derived FADGDH by linking the cytochrome molecule to the N terminus.

### Example 2: Fusion Protein Comprising Fungus-Derived FADGDH and Cytochrome Molecule from Cellobiose Dehydrogenase

### (I) Construction of Fusion Protein

An examination of whether or not an enzyme having electron transfer ability could be constructed by linking the heme domain of fungal cellobiose dehydrogenase as a cytochrome molecule to the N-terminus of a fungus-derived FADGDH was made.

FIG. 6-1 illustrates the amino acid sequence of a fusion protein which is recombinantly produced based on a gene constructed by linking the cytochrome domain of cellobiose dehydrogenase from *Phanerochaete chrysosporium* (PcCDH heme) at the gene level to the N-terminus of a fungus-derived FADGDH in this Example. In addition, the "fungus-derived FADGDH" used in this Example is an FADGDH (AfGDH) from *Aspergillus flavus.*

It was aimed at constructing a desired fusion protein (PcCDH heme-AfGDH) by linking the cytochrome domain to the N-terminus of the FADGDH via the loop sequence of cellobiose dehydrogenase derived from *Phanerochaete chrysosporium* as a linker sequence. In addition, in this fusion protein, the cytochrome domain lacked the N-terminal signal sequence.

A fusion protein expression vector was constructed by inserting genes, which encode each fusion protein designed as described above, into the expression vector pPIC9 that causes homologous reconstitution on the genome. To the N-terminus of the gene encoding the cytochrome domain, the gene encoding the signal sequence of an α-factor derived from the expression vector was linked. Yeast (*Pichia pastoris*) was transformed with the obtained expression vector.

### (II) Gene Expression

### II-1. Method

Expression of the fusion protein in the transformants obtained by transformation with the expression vector was analyzed, and culture was performed using the transformants (sample No.3 and No.4) showing high activity. For culture, each transformant was inoculated into 3 mL BMGY medium (1% yeast extract, 2% peptone, 1.34% yeast nitrogen base (containing no amino acid), 0.002% biotin, 100 mM sodium phosphate buffer (pH 6.0), 1% glycerol) and cultured at 150 rpm at 30°C for 12 hours. Thereafter, the cells were inoculated into 50 mL BMGY medium and additionally cultured at 30°C at 150 rpm for 24 hours. After completion of culture, the cells were harvested (1500 g, 10 min, room temperature), suspended in BMMY medium (1% yeast extract, 2% peptone, 1.34% yeast nitrogen base (containing no amino acid), 0.002% biotin, 100 mM potassium phosphate buffer (pH 6.0), and induced in a 100-mL scale (at 30°C at 190 rpm for 72 hours). For induction, 5% methanol was added every 24 hours to a final concentration of 0.5%.

After completion of induction, the cells were harvested, and the obtained supernatant was subjected to ammonium sulfate precipitation (60% and 80% saturation). At 80% saturation with ammonium sulfate, the precipitate was dissolved in 20 mM potassium sulfate buffer (pH 6.5), and the obtained solution was dialyzed against 20 mM potassium phosphate buffer (pH 6.5) (2 hours at 4°C for the first time, 2 hours at 4°C for the second time, and overnight at 4°C for the third time). The solution after dialysis was used as a culture supernatant fraction. After dialysis, the solution was purified by anion exchange chromatography under the conditions of Resource Q column, Linear gradient 40CV 0-100 mM NaCl (20 mM potassium phosphate buffer (pH 6.5)).

After purification, the fraction was analyzed by SDS-PAGE, and the fraction including the target band was dialyzed against 20 mM potassium phosphate buffer (pH 6.5) (overnight at 4°C) to obtain a purified enzyme.

The wet cells were lysed with a French press, centrifuged (at 13,000g at 4°C for 20 minutes), and then ultracentrifuged (at 108, 000g at 4°C for 1 hour). The supernatant was dialyzed to obtain a water-soluble fraction. The obtained water-soluble fraction and culture supernatant fraction were subjected to SDS-PAGE analysis as described above.

### II-2. Results and discussion

FIG. 7 illustrates the results of SDS-PAGE analysis. Compared to the culture supernatant (OS) of non-transformed host cells (*P. pastoris*), in samples No. 3 and No. 4, a smeared band was observed around 100 to 120 kDa. The predicted molecular weight of the fusion protein (PcCDH heme-AfGDH) of this Example was about 83 kDa, and this result suggests that a band shift due to sugar chain modification occurred.

### (III) Evaluation of enzymatic activity

### III-1. Method

The GDH activity of the obtained fusion protein was evaluated using the following systems.
- DCIP (0.06 mM (final concentration))/PMS (0.6 mM (final concentration)) system: for evaluation, 20 mM potassium phosphate buffer (pH 6.5) was used as a buffer, and glucose (5, 10, 20, 50, 100, and 200 mM (final concentrations)) was used as a substrate.
- Ru ((w/v) 2% Ru (III))/MTT (1 mM MTT (final concentration)) system: for evaluation, 20 mM potassium phosphate buffer (pH 6.5) was used as a buffer, and glucose (20 and 200 mM (final concentrations)) was used as a substrate.
- MTT (1 mM MTT (final concentration)) system: for evaluation, 20 mM potassium phosphate buffer (pH 6.5) was used as a buffer, and glucose (20 and 200 mM (final concentrations)) was used as a substrate.

For evaluation, the activity of the enzyme that oxidizes 1 µmol of glucose for 1 minute was defined as 1 unit.

### III-2. Results

The Kₘ and Vₘₐₓ of the obtained PcCDH heme-AfGDH were calculated from the Lineweaver-Burk plot, and as a result, the Kₘ was 53 mM and the Vₘₐₓ was 250 U/mg. Compared to AfGDH produced recombinantly in *E. coli* (Kₘ = 45.8 mM, and Vₘₐₓ = 400 U/mg), it was confirmed that the Vₘₐₓ was reduced to 63%. This is considered to be due to sugar chain modification or the linking of the cytochrome domain to the N-terminus.

In addition, almost no activity was observed in the Ru/MTT system (200 mM glucose: 2.0×10⁻³ U/mg; 20 mM glucose: 3.2×10⁻³ U/mg).

In addition, the MTT system showed an activity equal to about 1/1000 of the PMS/DCIP system, but showed a 10-fold activity compared to the Ru/MTT system. Furthermore, when Ru was added after the evaluation of the activity in the MTT system, the reaction greatly decreased.

These results suggest that Ru functions as an inhibitor and the efficiency of electron transfer from the FAD of AfGDH to the heme of cytochrome domain is low. Furthermore, when the substrate concentration in the MTT system was changed, the maximum activity could be obtained for 20 mM glucose, and when the substrate concentration was high, a decrease in the activity was observed. This suggests that substrate inhibition occurs at a concentration of 20 mM glucose or higher in the MTT system.

### (IV) Measurement of UV-VIS spectrum

### IV-1. Method

Glucose (final concentration: 20 mM) was added to the obtained PcCDH heme-AfGDH in 20 mM potassium phosphate buffer (pH 6.5), and after 0, 1, 5, 10, 30,60, 120 and 180 minutes, the absorption spectrum at 400 to 600 nm was measured.

### IV-2. Results

FIG. 8 illustrates the results of measurement of the spectrum, and the absorption spectrum of the solet band (420 to 430 nm) derived from the heme was observed. In addition, after the addition of glucose, a shift of the peak to 432 nm over 0 to 180 minutes was observed. In addition, reduction peaks of the cytochrome domain were observed at 530 nm and 560 nm.

From these results, it was confirmed that electron transfer within the molecule of the fusion protein takes place

FIG. 9 illustrates the spectral difference (the value of reduction by glucose addition - the value of oxidation) in the UV-VIS spectral measurement between before and after addition of glucose. The reduction peak of the cytochrome domain was clearly observed. From this, it was confirmed that, after glucose was added and oxidized and the FAD of AfGDH was reduced by the addition of glucose, electrons were transferred from the reduced FAD to the cytochrome domain and the cytochrome domain was reduced.

### (V) Electrochemical evaluation of DET ability

### V-1. Method

0.8 µL of a 2% (w/w) MWNT (MW-I, Meijo Nano Carbon Co., Ltd.) dispersion was applied once or twice onto the CE (4.8 mm²) of a carbon printed electrode (DEP chip) and dried. Furthermore, 1.0 µL of 10 mM 1-pyrenebutyric acid N-hydroxysuccinimide ester (PyNHS in DMF) was applied to the applied MWNT and dried. Thereafter, 3 µL of 5 mg/mL PcCDH heme-AfGDH in 20mM potassium phosphate buffer (pH 7.5) was dropped onto the MWNT/PyNHS-applied chip and allowed to stand at 25°C for 2 hours under high humidity, and then the enzyme solution on the chip was naturally dried. After drying, crosslinking was performed at 25°C for 30 minutes under a glutaraldehyde atmosphere. The fabricated electrode chip was stored under low humidity (McDry: 1% RH) until use. For measurement, the electrode chip was washed with a 100 mM potassium phosphate buffer (pH 7.0) for 20 minutes, and then electrochemical measurement was performed.

The electrochemical measurement was performed in a batch cell (2.0 mL in 5 mL volume cell) at room temperature (25°C ± 2°C) using the PcCDH heme-AfGDH-immobilized electrode as a working electrode, a platinum electrode as a counter electrode, and an Ag/AgCl electrode (BAS RE-1 B) electrode as a reference electrode.

The CA measurement was performed at a stirring speed of 300 rpm in 100 mM potassium phosphate buffer (pH 6.0, 6.5 and 7.0). As for the calibration curve, a potential of +400 mV vs. Ag/AgCl was applied, and the response current was measured by adding a glucose solution, prepared in the same buffer as that for the measurement solution, sequentially at 0.1, 0.5, 1, 2.5, 5, 10, 20 and 50 mM in order. After the measurement, CA measurement was performed using the same chip in the measurement solution (50 mM glucose, 100 mM potassium phosphate buffer (pH 6.0)) at an applied potential of +400 mV.

### V-2. Results

FIG. 10 illustrates the results of CA measurement in the measurement solution at pH 6.0, and a clear response by the addition of the substrate could be confirmed. When the enzyme concentration at the time of immobilization was low and the pH of the measurement solution was 6.5, almost no response was observed (FIGS. 10(A) and 10(B)). Furthermore, the measurement solution was replaced and the pH value was changed in the order of (1) pH 6, (2) pH 7, and (3) pH 6, and then the current value was measured, and as a result, no response was observed at pH 7 (FIG. 10(C)). From this, it was suggested that, even in the fusion with AflGDH, electron transfer to PcCDH heme is pH-dependent, and that the electron transfer occurs at a low pH.

As described above, the fungus-derived FADGDH having direct electron transfer ability could be obtained by linking the heme domain of fungal cellobiose dehydrogenase as a cytochrome molecule to the N-terminus of FADGDH. Furthermore, it was confirmed that the FADGDH may be used as an element for a glucose sensor having direct electron transfer ability, which does not require an electron mediator.

### Example 3: Fusion Protein Comprising Fungus-Derived FADGDH Variant and Cytochrome Molecule from Cellobiose Dehydrogenase

### (I) Construction of fusion protein

An examination of whether or not an enzyme having direct electron transfer ability could be constructed by linking the heme domain of fungal cellobiose dehydrogenase as a cytochrome molecule to the N-terminus of a fungus-derived FADGDH variant was made.

FIGS. 6-2, 6-3, 6-4 and 6-5 illustrate the amino acid sequences of fusion proteins which are each produced recombinantly based on a gene constructed by linking the cytochrome domain of cellobiose dehydrogenase from *Phanerochaete chrysosporium* at the gene level to the N-terminus of a fungus-derived FADGDH variant in this Example. In addition, the "fungus-derived FADGDH" used in this Example is an FADGDH (AfGDH) derived from *Aspergillus flavus.*

It was aimed at constructing a desired fusion protein (PcCDH heme-AfGDH variant) by linking the cytochrome domain to the N-terminus of the FADGDH variant via the loop sequence of cellobiose dehydrogenase derived from *Phanerochaete chrysosporium* as a linker sequence. In addition, this fusion protein lacks the N-terminal signal sequence of the cytochrome domain.

A fusion protein expression vector was constructed by inserting genes, which encode each variant fusion protein designed as described above, into the expression vector pPIC9 that causes homologous reconstitution on the genome. To the N-terminus of the gene encoding the cytochrome domain, the gene encoding the signal sequence of an α-factor derived from the expression vector was linked. Yeast (*Pichia pastoris*) was transformed with the obtained expression vector.

### (II) Gene expression

### II-1. Method

Expression of the fusion protein in the transformants obtained by transformation with the expression vector was analyzed, and culture was performed using the transformants showing high activity. For culture, each transformant was inoculated into 3 mL BMGY medium (1% yeast extract, 2% peptone, 1.34% yeast nitrogen base (containing no amino acid), 0.002% biotin, 100 mM sodium phosphate buffer (pH 6.0), 1% glycerol) and cultured at 150 rpm at 30°C for 12 hours. Thereafter, the cells were inoculated into 50 mL BMGY medium and additionally cultured at 30°C at 150 rpm for 24 hours. After completion of culture, the cells were harvested (1500 g, 10 min, room temperature), suspended in BMMY medium (1% yeast extract, 2% peptone, 1.34% yeast nitrogen base (containing no amino acid), 0.002% biotin, 100 mM potassium phosphate buffer (pH 6.0)), and induced in a 100-mL scale (at 30°C at 190 rpm for 72 hours). For induction, 5% methanol was added every 24 hours to a final concentration of 0.5%.

After completion of induction, the cells were harvested, and the obtained supernatant was subjected to ammonium sulfate precipitation (60% and 80% saturation). At 80% saturation with ammonium sulfate, the precipitate was dissolved in 20 mM potassium sulfate buffer (pH 6.5), and the obtained solution was dialyzed against 20 mM potassium phosphate buffer (pH 6.5) (2 hours at 4°C for the first time, 2 hours at 4°C for the second time, and overnight at 4°C for the third time). The solution after dialysis was used as a culture supernatant fraction. After dialysis, the solution was purified by anion exchange chromatography under the conditions of Resource Q column, Linear gradient 40CV 0-100 mM NaCl (20 mM potassium phosphate buffer (pH 6.5)).

After purification, the fraction was analyzed by SDS-PAGE, and the fraction including the target band was dialyzed against 20 mM potassium phosphate buffer (pH 6.5) (overnight at 4°C) to obtain a purified enzyme.

The wet cells were lysed with a French press, centrifuged (at 13,000g at 4°C for 20 minutes), and then ultracentrifuged (at 108, 000g at 4°C for 1 hour). The supernatant was dialyzed to obtain a water-soluble fraction. The enzymatic activities of the obtained water-soluble fraction and culture supernatant fraction were measured.

### (III) Evaluation of enzymatic activity

### III-1. Method

The GDH activity of the obtained variant fusion protein was evaluated using the following systems.
- DCIP (0.06 mM (final concentration))/PMS (0.6 mM (final concentration)) system: for evaluation, 20 mM potassium phosphate buffer (pH 6.5) was used as a buffer, and glucose (5, 10, 20, 50, 100 and 200 mM (final concentrations)) was used as a substrate.
- Ru ((w/v) 2% Ru (III))/MTT (1 mM MTT (final concentration)) system: for evaluation, 20 mM potassium phosphate buffer (pH 6.5) was used as a buffer, and glucose (20 and 200 mM (final concentrations)) was used as a substrate.
- MTT (1 mM MTT (final concentration)) system: for evaluation, 20 mM potassium phosphate buffer (pH 6.5) was used as a buffer, and glucose (20 and 200 mM (final concentrations)) was used as a substrate. For evaluation, the activity of the enzyme that oxidizes 1 µmol of glucose for 1 minute was defined as 1 unit.

### III-2. Results

The enzymatic activities of the obtained PcCDH heme-AfGDH (variants) were measured. As a result, fusion enzymes of AfGDH variants, including Glu346Lys (E346K), Glu346Arg (E346R), Asn430Lys (N430K), Asn430Arg (N430R), Thr498Lys (T498K), Thr498Arg (T498R), Glu504Lys (E504K), and Glu504Arg (E504R), all achieved enzyme production equivalent to or higher than that of the wild-type enzyme (Table 2).

**[Table 2]**

| | PMS/DCIP (U/L culture) | MTT (U/L culture) | | PMS/DCIP (U/L culture) | MTT (U/L culture) |
|---|---|---|---|---|---|
| WT | 12576.7 | 2.2 | | | |
| E346K | 13441.7 | 0.15 | E346R | 11693.3 | 0.1 |
| N430K | 17049.1 | 0.2 | N430R | 19699.4 | 0.4 |
| T498K | 12374.2 | 0.15 | T498R | 8914.1 | 0.1 |
| E504K | 13674.8 | 0.15 | E504R | 14429.5 | 0.35 |

Thus, as a representative of the variants at each position, Glu346Lys (E346K), Asn430Lys (N430K), Thr498Lys (T498K) and Glu504Lys (E504K), in which each residue was substituted with Lys, were purified by anion exchange chromatography and evaluated in detail.

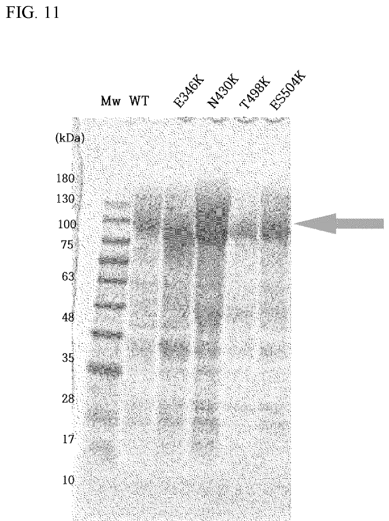

The enzyme samples prepared as described above were subjected to SDS-PAGE analysis, and as a result, it was confirmed that these variant fusion enzymes all had the same molecular weight as that of the wild-type AfGDH, and were clearly recovered as fusion proteins (FIG. 11).

The Kₘ and Vₘₐₓ of the variant fusion enzymes were calculated from the Lineweaver-Burk plot, and as a result, it was observed that these variant enzymes all had higher activity than the fusion enzyme constructed based on the wild-type FADGDH.

### (IV) Measurement of UV-VIS spectrum

### IV-1. Method

Glucose (final concentration: 20 mM) was added to the obtained PcCDH heme-AfGDH (variant) in 20 mM potassium phosphate buffer (pH 6.5), and after 0, 1, 5, 10, 30,60, 120 and 180 minutes, the absorption spectrum at 400 to 600 nm was measured.

### IV-2. Results

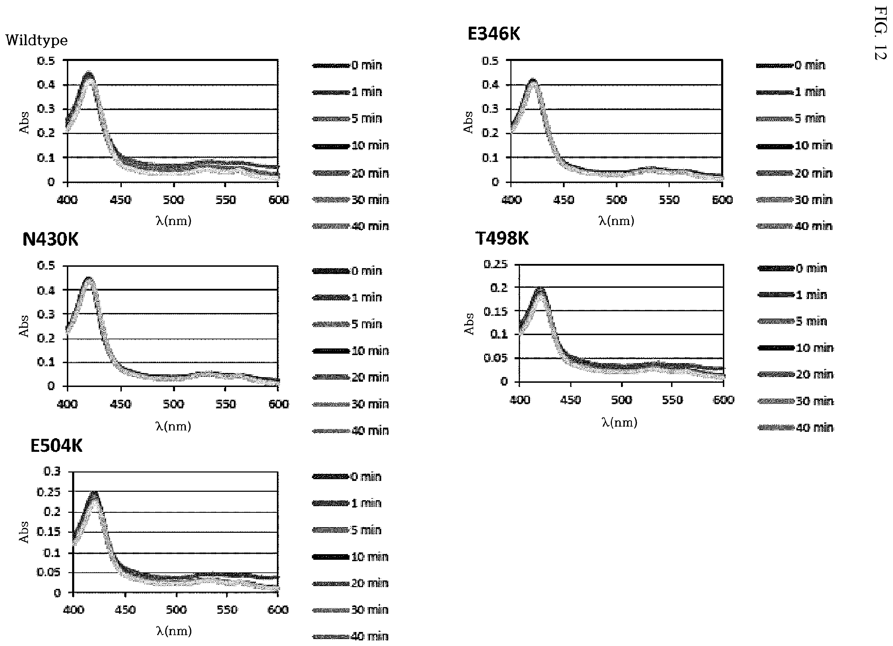

FIG. 12 illustrates the results of measurement of the spectrum, and the absorption spectrum of the solet band (420 to 430 nm) derived from the heme was observed. In addition, after the addition of glucose, a shift of the peak to 432 nm over 0 to 180 minutes was observed. In addition, reduction peaks of the cytochrome domain were observed at 530 nm and 560 nm.

From these results, it was confirmed that electron transfer within the molecule of the variant fusion protein takes place.

### (V) Electrochemical evaluation of DET ability

### V-1. Method

0.8 µL of a 2% (w/w) MWNT (MW-I, Meijo Nano Carbon Co., Ltd.) dispersion was applied onto the CE (4.8 mm²) of a carbon printed electrode (DEP chip) and dried. Furthermore, 1.0 µL of 10 mM 1-pyrenebutyric acid N-hydroxysuccinimide ester (PyNHS in DMF) was applied to the applied MWNT and dried. Thereafter, 0.25 U of the wild-type fusion protein or 0.1 U of the variant fusion protein (Glu346Lys (E346K), Asn430Lys (N430K), Thr498Lys (T498K), or Glu504Lys (E504K)), prepared in 20 mM potassium phosphate buffer (pH 7.5), was dropped onto the MWNT/PyNHS-applied chip and allowed to stand at 25°C for 2 hours under high humidity, and then the enzyme solution on the chip was naturally dried. The fabricated electrode chip was stored under low humidity (McDry: 1% RH) until use. For measurement, the electrode chip was washed with 100 mM potassium phosphate buffer (pH 7.0) for 20 minutes, and then electrochemical measurement was performed.

Electrochemical measurement was performed in a batch cell (2.0 mL in 5 mL volume cell) at room temperature (25°C ± 2°C) using the PcCDH heme-AfGDH (variant)-immobilized electrode as a working electrode, a platinum electrode as a counter electrode, and an Ag/AgCl electrode (BAS RE-1 B) electrode as a reference electrode.

CA measurement was performed at a stirring speed of 300 rpm in 100 mM potassium phosphate buffer (pH 6.0, 6.5 and 7.0). As for the calibration curve, a potential of +400 mV vs. Ag/AgCl was applied, and the response current was measured by adding a glucose solution, prepared in the same buffer as that for the measurement solution, sequentially at 0.1, 0.5, 1, 2.5, 5, 10, 20 and 50 mM in order. After the measurement, CA measurement was performed using the same chip in the measurement solution (50 mM glucose, 100 mM potassium phosphate buffer (pH 6.0)) at an applied potential of +400 mV.

### V-2. Results

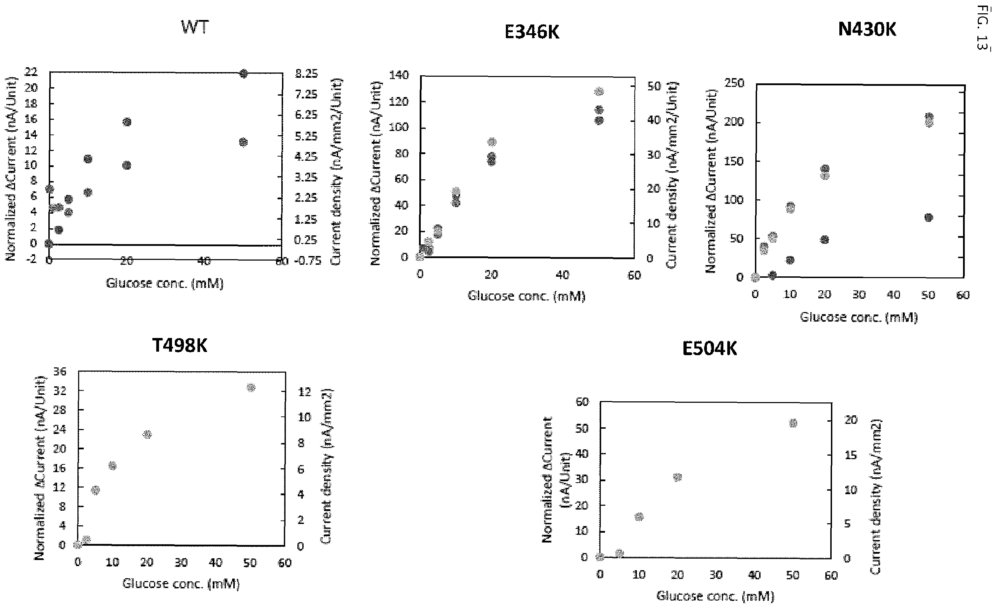

FIG. 13 illustrates the results of CA measurement in the measurement solution at pH 6.0, and a clear response by the addition of the substrate could be confirmed. The constructed fusion proteins, comprising each of the AfGDH variants (Glu346Lys (E346K), Asn430Lys (N430K), Thr498Lys (T498K), and Glu504Lys (E504K)), all exhibited direct electron transfer ability. In addition, the variant fusion proteins, including Glu346Lys (E346K), Asn430Lys (N430K), Thr498Lys (T498K) and Glu504Lys (E504K), exhibited the response based on direct electron transfer at a higher current density than the fusion protein comprising the wild-type AfGDH. From this, it was suggested that these variant fusion proteins had improved direct electron transfer ability.

As described above, the FADGDH variant fusion protein having improved direct electron transfer ability compared to the wild-type protein could be obtained by linking the heme domain of fungal cellobiose dehydrogenase as a cytochrome molecule to the N-terminus of each of the FADGDH variants (Glu346Lys (E346K), Asn430Lys (N430K), Thr498Lys (T498K), and Glu504Lys (E504K)). In addition, it was confirmed that this FADGDH variant fusion protein may be used an element for a glucose sensor having direct electron transfer ability, which does not require an electron mediator.

## Claims

1. A fusion protein comprising a fungus-derived flavin adenine dinucleotide glucose dehydrogenase (FADGDH) and a cytochrome molecule linked to the N-terminus of the FADGDH, which is one of the following (a) to (b):
(a) a protein which consists of an amino acid sequence set forth in at least one of SEQ ID NOs: 2, 3 and 13 to 21; and
(b) a protein which consists of an amino acid sequence having a sequence identity of 85% or higher to the amino acid sequence according to (a), and which has glucose dehydrogenase activity and electron transfer function.

2. A gene encoding the fusion protein set forth in claim 1.

3. A vector comprising the gene set forth in claim 2.

4. A transformant comprising the gene set forth in claim 2.

5. A transformant in which the gene set forth in claim 2 is integrated into the main chromosome of the transformant.

6. An enzyme electrode comprising the fusion protein set forth in claim 1.

7. A method for measuring glucose concentration in a sample, the method comprising:
bringing the sample into contact with the enzyme electrode set forth in claim 6; and
measuring electrons generated by oxidation of glucose.

8. A glucose sensor which uses the enzyme electrode set forth in claim 6 as a working electrode.

## Patentansprüche

1. Fusionsprotein, das eine aus Fungus stammende Flavin-Adenin-Dinucleotid-Glucose-Dehydrogenase (FADGDH) und ein an den N-Terminus der FADGDH gebundenes Cytochrom-Molekül umfasst,
das eines der folgenden (a) bis (b) ist:
(a) ein Protein, das aus einer Aminosäuresequenz besteht, die in mindestens einer von SEQ ID NOs: 2, 3 und 13 bis 21 dargestellt ist; und
(b) ein Protein, das aus einer Aminosäuresequenz besteht, die eine Sequenzidentität von 85% oder höher mit der Aminosäuresequenz nach Anspruch (a) aufweist, und das eine Glucose-Dehydrogenase-Aktivität und eine Elektronentransferfunktion aufweist.

2. Gen, das das in Anspruch 1 aufgezeigte Fusionsprotein codiert.

3. Vektor, der das in Anspruch 2 aufgezeigte Gen umfasst.

4. Transformante, die das in Anspruch 2 aufgezeigte Gen umfasst.

5. Transformante, bei der das in Anspruch 2 aufgezeigte Gen in das Hauptchromosom der Transformante integriert ist.

6. Enzymelektrode, die das in Anspruch 1 aufgezeigte Fusionsprotein umfasst.

7. Verfahren zum Messen der Glucosekonzentration in einer Probe, wobei das Verfahren umfasst:
Inkontaktbringen der Probe mit der in Anspruch 6 aufgezeigten Enzymelektrode; und Messen der durch Oxidation von Glucose erzeugten Elektronen.

8. Glucose-Sensor, der die in Anspruch 6 aufgezeigte Enzymelektrode als Arbeitselektrode verwendet.

## Revendications

1. Protéine de fusion comportant une glucose déshydrogénase dépendante du flavine-adénine dinucléotide (FADGDH) dérivée d'un champignon et une molécule de cytochrome liée à l'extrémité N terminale du FADGDH, qui est l'un des éléments suivants (a) et (b) :
(a) une protéine qui consiste en une séquence d'acides aminés présentée dans au moins l'un des SEQ ID NO : 2, 3 et 13 à 21 ; et
(b) une protéine qui consiste en une séquence d'acides aminés ayant une identité de séquence de 85 % ou plus par rapport à la séquence d'acides aminés selon (a), et qui a une activité de glucose déshydrogénase et une fonction de transfert d'électrons.

2. Gène codant pour la protéine de fusion présentée dans la revendication 1.

3. Vecteur comportant le gène présenté dans la revendication 2.

4. Transformant comportant le gène présenté dans la revendication 2.

5. Transformant dans lequel le gène présenté dans la revendication 2 est intégré dans le chromosome principal du transformant.

6. Électrode enzymatique comportant la protéine de fusion présentée dans la revendication 1.

7. Procédé de mesure d'une concentration en glucose dans un échantillon, le procédé comportant :
la mise en contact de l'échantillon avec l'électrode enzymatique présentée dans la revendication 6 ; et
la mesure des électrons générés par oxydation de glucose.

8. Capteur de glucose qui utilise l'électrode enzymatique présentée dans la revendication 6 comme électrode de travail.
